(19) Europäisches Patentamt
European Patent Office
Office européen des brevets

(11) Numéro de publication : **0 477 053 B1**

# (12) FASCICULE DE BREVET EUROPEEN

(45) Date de publication du fascicule du brevet : **12.01.94 Bulletin 94/02**

(51) Int. Cl.$^5$ : **A61K 7/00,** A61K 7/06, A61K 7/48

(21) Numéro de dépôt : **91402302.3**

(22) Date de dépôt : **23.08.91**

## (54) Composition cosmétique pour cheveux contenant un polymère filmogène et une silicone incorporés dans une microdispersion de cire, et procédé de traitement cosmétique.

(30) Priorité : **23.08.90 FR 9010602**

(43) Date de publication de la demande : **25.03.92 Bulletin 92/13**

(45) Mention de la délivrance du brevet : **12.01.94 Bulletin 94/02**

(84) Etats contractants désignés : **AT BE CH DE DK ES FR GB GR IT LI NL SE**

(56) Documents cités :
**EP-A- 0 167 825**
**EP-A- 0 206 128**
**EP-A- 0 374 332**
**EP-A- 0 394 078**
**FR-A- 2 232 303**
**US-A- 4 551 330**

(73) Titulaire : **L'OREAL**
**14, Rue Royale**
**F-75008 Paris (FR)**

(72) Inventeur : **Grollier, Jean-François**
**16bis, Boulevard Morland**
**75004 Paris (FR)**
Inventeur : **Rellet, Isabelle**
**6, rue Ordener**
**75018 Paris (FR)**
Inventeur : **Peritz, Lyonnel**
**3bis, rue du Mont Valérien**
**92210 St. Cloud (FR)**

(74) Mandataire : **Tonnellier, Jean-Claude et al**
**Cabinet Nony & Cie. 29, rue Cambacérès**
**F-75008 Paris (FR)**

EP 0 477 053 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Composition cosmétique pour cheveux contenant un polymère filmogène et une silicone incorporés dans une microdispersion de cire, et procédé de traitement cosmétique.

La présente invention a pour objet une composition cosmétique pour cheveux contenant un polymère filmogène et une silicone incorporés dans une microdispersion de cire, ainsi qu'un procédé de traitement cosmétique des cheveux à l'aide d'une telle composition.

On sait que de nombreuses compositions cosmétiques capillaires contiennent un polymère filmogène qui, appliqué sous la forme d'une solution, permet la formation, après évaporation du solvant, d'un film gainant les cheveux. Il est connu que les polymères filmogènes utilisés dans les compositions cosmétiques pour cheveux ont en commun notamment la propriété de renforcer la tenue de la coiffure.

Il est par ailleurs connu d'incorporer dans les compositions cosmétiques pour cheveux des silicones qui présentent notamment la propriété d'améliorer la douceur des cheveux.

On sait par ailleurs qu'il est possible d'obtenir avec certaines huiles des microémulsions et avec certaines cires des microdispersions stables et diluables à l'eau indéfiniment, sans agrégation ni sédimentation des particules en suspension. Les microdispersions de cire sont obtenues par fusion de la cire en présence d'un tensioactif anionique ou non-ionique, et éventuellement d'une partie de l'eau, puis addition progressive d'eau chaude avec agitation. On observe la formation intermédiaire d'une émulsion du type eau-dans-l'huile, suivie d'une inversion de phase avec obtention finale d'une émulsion du type huile-dans-l'eau. Au refroidissement, on obtient une microdispersion stable de particules colloïdales solides de cire ; voir par exemple "Microemulsions Theory and Practice", L.M. Prince Ed., Academic Press (1977) pages 21-32.

Jusqu'à présent ces microdispersions de cire étaient utilisées notamment pour faire briller les articles en cuir et les revêtements de sol en matière plastique.

Dans la demande de brevet européen n°90 400515 déposée le 23/02/1990 et intitulé "Utilisation, comme composition cosmétique pour cheveux, d'une microdispersion de cire, et procédé de traitement des cheveux avec une telle composition", on a décrit l'utilisation, comme composition cosmétique ou support de composition cosmétique pour cheveux, d'une composition fluide constituée essentiellement par une microdispersion stable de cire dans un véhicule liquide aqueux.

On a maintenant découvert que, de façon surprenante, des compositions cosmétiques pour cheveux renfermant un polymère filmogène et une silicone dans une microdispersion de cire confèrent de bonnes propriétés de douceur des cheveux et de tenue de la coiffure, alors que les compositions renfermant seulement la microdispersion de cire et une silicone diminuent la tenue de la coiffure et que les compositions renfermant seulement la microdispersion de cire et le polymère filmogène diminuent la douceur de la chevelure.

La présente invention a donc pour objet une composition cosmétique pour cheveux comprenant au moins un polymère filmogène et au moins une silicone incorporés dans un support constitué essentiellement d'une dispersion de cire dans un véhicule liquide aqueux dont la phase dispersée est une microdispersion stable de particules de dimensions inférieures à 500 nm, lesdites particules étant constituées essentiellement d'une cire ou d'un mélange de cires, ladite cire ou ledit mélange de cires ayant un point de fusion finissante supérieur à 60°C et inférieur à 100°C et étant capable de former une microdispersion telle que définie ci-dessus, ladite composition contenant, en poids, de 0,1 à 40 % de cire et une quantité suffisante d'au moins un agent émulsionnant.

On sait que les cires sont des substances naturelles (animales ou végétales) ou synthétiques, solides à la température ordinaire (21°C), ayant généralement une certaine plasticité, qui sont insolubles dans l'eau, solubles dans les huiles, et qui sont capables de former des films hydrofuges. Sur la définition des cires et leurs utilisations en cosmétologie, on peut citer par exemple P.D. Dorgan, Drug and Cosmetic Industry, Décembre 1983, pages 30-33, et Handbook of Cosmetic Science, H.W. Hibbot ed., Pergamon Press, Oxford (1963) page 60.

La cire ou le mélange de cires utilisé dans la composition selon l'invention doit être capable de donner, en association avec des agents émulsionnants, notamment non ioniques et/ou anioniques, selon le procédé décrit ci-dessus, des microdispersions stables ayant des dimensions de particules inférieures à 500 nm. Les cires ou mélanges de cires utilisables peuvent être choisis par de simples expériences de routine.

La cire est notamment une cire choisie parmi la cire de Carnauba, la cire de Candelilla, la cire d'Alfa et leurs mélanges ; la cire peut contenir, outre les cires dejà citées une autre cire ou un mélange d'autres cires, par exemple une cire de paraffine ; la proportion pondérale de cire de Carnauba et/ou de Candelilla et/ou d'Alfa, dans de tels mélanges, est de préférence supérieure ou égale à 50%.

Les cires végétales de Carnauba (extraite de Copernicia Cerifera), de Candelilla (extraite de Euphorbies Cerifera et de Pedilantus Pavonis), et d'Alfa (extraite de Stipa Tenacissima) sont des produits commerciaux.

Il est également possible selon l'invention d'utiliser les céramides, notamment en association avec au moins une des cires mentionnées ci-dessus.

Les céramides sont les principaux lipides constitutifs des espaces intercornéocytaires du stratum corneum. Ils ont été décrits, en particulier par Downing dans Science 1982 p 1261-2 vol.18. Les céramides ont déjà été utilisés notamment dans des compositions cosmétiques pour cheveux : voir par exemple la demande de brevet européen 0278505.

Ces céramides sont généralement difficiles à disperser dans les compositions cosmétiques. Dans les compositions de la présente invention, il est possible de les disperser facilement.

La proportion de cires, dans la composition, est par exemple de 0,1 à 20 %, notamment de 1 à 15 %, et en particulier de 1 à 10%.

La proportion pondérale de cire dans les particules de la microdispersion est généralement supérieure à 90%, par rapport au poids des particules, et est le plus souvent supérieure à 95%, le reste étant constitué par les ingrédients liposolubles éventuellement présents (non compris les agents émulsionnants).

Le rapport pondéral cire/émulsionnant peut varier dans la gamme de 1 à 20, notamment de 2 à 10.

Il est également possible de préparer des microdispersions de cires en utilisant des mélanges commerciaux de cires auto-émulsionnables contenant la cire et les émulsionnants. On peut utiliser par exemple la cire commercialisée sous la dénomination CIRE AUTO LUSTRANTE OFR par TISCCO, Bobigny (France), qui contient des cires de Carnauba et de paraffine, en association avec des agents émulsionnants non ioniques, ou la cire auto-émulsionnable commercialisée sous la dénomination CERAX A.O. 28/B par LA CERESINE, Marseille (France), qui contient de la cire d'Alfa en association avec un émulsionnant non ionique. Ces mélanges commerciaux permettent de préparer des microdispersions de cires par addition d'eau selon le procédé décrit ci-dessus.

L'agent émulsionnant utilisé pour permettre la préparation de microdispersions stables de cire, telles que définies ci-dessus, est de préférence un agent tensio-actif anionique ou non ionique. La concentration de l'agent émulsionnant est au moins égale à la concentration nécessaire pour obtenir une telle microdispersion. Cette concentration minimum peut être déterminée dans chaque cas par de simples espériences de routine. Généralement, elle peut varier de 0,01 à 25 % en poids, par rapport au poids total de la composition, et en particulier de 0,1 à 10 % en poids.

Les tensio-actifs anioniques utilisés ont de préférence une balance lipophile-hydrophile (HLB) pouvant aller de 10 à 40. Ce sont notamment les sels d'acides gras (par exemple sels alcalins ou sels organiques tels que les sels d'amines), lesdits acides gras ayant par exemple de 12 à 18 atomes de carbone et pouvant comporter une double liaison comme dans le cas de l'acide oléique; les sels alcalins ou sels de bases organiques des acides alkyl-sulfuriques et alkyl-sulfoniques ayant 12 à 18 atomes de carbone, des acides alkyl-arylsulfoniques dont la chaîne alkyle contient de 6 à 16 atomes de carbone, le groupement aryle étant par exemple un groupement phényle. Ce sont également les éthers-sulfates, en particulier les produits de sulfatation des alcools gras et alkylphénols polyalcoxylés, dans lesquels la chaîne aliphatique comporte de 6 à 20 atomes de carbone et la chaîne polyalcoxylée de 1 à 30 motifs oxyalkylène, en particulier oxyéthylène, oxypropylène ou oxybutylène.

Tous ces tensio-actifs anioniques sont bien connus et beaucoup d'entre eux sont des produits commerciaux.

Les tensio-actifs non ioniques sont principalement des tensio-actifs polyalcoxylés et/ou polyglycérolés . Ce sont notamment les acides gras ou les amides d'acide gras polyalcoxylés et/ou polyglycérolés ; les esters d'acides gras et de polyols polyalcoxylés et/ou polyglycérolés ; les alcools gras ou les alkylphénols polyalcoxylés et/ou polyglycérolés; les alcanediols ou alcènediols-1,2 ou -1,3 polyalcoxylés et/ou polyglycérolés ; et les alkyléthers d'alcanediols ou alcènediols -1,2 ou -1,3 polyalcoxylés et/ou polyglycérolés. Les acides ou alcools gras, éventuellement insaturés, ont par exemple 12 à 24 atomes de carbone, la chaîne alkyle des alkylphénols a par exemple 6 à 16 atomes de carbone, les alcanediols ou alcènediols ont de 9 à 24 atomes de carbone, l'alkyle des alkyléthers a de 4 à 20 atomes de carbone, et le nombre de motifs oxyalkylène ou de motifs ($CH_2CHOH$ $CH_2O$) peut aller de 2 à 40.

Les dérivés non ioniques polyalcoxylés sont notamment des dérivés polyoxyéthylénés, éventuellement polyoxypropylénés.

Les acides gras polyalcoxylés sont des produits commerciaux, notamment les produits vendus sous la marque Myrj par la Société ATLAS).

Les esters d'acides gras et de polyols polyoxyéthylénés pour lesquels le polyol est le sorbitol sont des produits connus (Polysorbate et produits commercialisés sous la marque Tween par la Société ATLAS).

Les alcools gras polyoxyéthylénés sont des produits commerciaux, notamment ceux vendus sous la marque Brij par la Société ATLAS.

Les alcools gras polyglycérolés, les alcanediols ou alcènediols polyglycérolés, ou les alkyléthers d'alcanediols ou d'alcènediols polyglycérolés peuvent être préparés par exemple selon les procédés décrits dans les brevets français 1.477.048, 2.025.681, 2.091.516 et 2.465.780, ou selon des procédés analogues.

Les acides gras ou amides d'acides gras polyglycérolés sont notamment décrits dans le brevet français 1.484.723 ou sont encore des produits commerciaux tels que ceux vendus sous la marque PLUROL (Gattefossé) ou DREWPOL (Stefan Company).

Comme indiqué ci-dessus, la phase continue de la microdispersion est une phase aqueuse. Généralement la composition de l'invention contient, en poids, au moins 35 % d'eau. La proportion d'eau dans la phase liquide varie généralement de 80 à 100 % par rapport au poids de la phase liquide.

Les silicones présentes dans la composition de l'invention sont des polyorganosiloxanes. Il s'agit de produits connus qui peuvent se présenter sous forme d'huiles, de gommes, de résines ou de cires. Dans ces trois derniers cas, elles peuvent être ajoutées à la composition sous la forme d'une solution dans un solvant organique, ou en mélange avec des silicones liquides dans lesquelles elles sont solubles.

D'une façon générale, les silicones sont des polymères contenant des motifs de formule brute I :

$$\left[ R_n \; SiO_{\frac{4-n}{2}} \right] \quad (I)$$

Ces polymères contiennent des motifs répétitifs qui sont les motifs de formule I pour lesquels n = 2. Les substituants R présents dans ces motifs répétitifs sont des groupements organiques. Les groupements R liés à un même atome de silicium peuvent être identiques ou différents. En outre, une même molécule polymère peut contenir des motifs répétitifs différents.

Les motifs répétitifs correspondant à n = 2 confèrent à la molécule polymère une structure linéaire ou cyclique dont la chaîne est constituée de liaisons siloxane. Dans le cas d'un polymère linéaire, des motifs correspondant à n = 3 constituent les groupements terminaux.

En outre, les polyorganosiloxanes peuvent contenir des motifs réticulants intercalés entre les motifs répétitifs. Ces motifs réticulants correspondent à la formule I avec n = 1 ou n = 0.

Dans les motifs répétitifs (n = 2) et les motifs réticulants correspondant à n = 1, les groupements R peuvent représenter notamment des groupements alkyle, cycloalkyle ou aryle et peuvent comporter en outre, des groupements fonctionnels (éthers, amines, carboxyles, hydroxyles, thiols, esters, sulfonates, sulfates, etc.). Les groupements alkyle ont par exemple 1 à 20 atomes de carbone ; les groupements cycloalkyle ont par exemple 5 ou 6 chaînons ; les groupements aryle sont notamment des groupements phényle.

Dans le cas des groupements terminaux correspondant à n = 3, l'un des groupements R attachés au silicium terminal peut en outre représenter un autre groupement tel qu'un groupement OH.

Les silicones qui répondent à la définition donnée ci-dessus sont des composés connus.

La teneur pondérale en silicones peut varier généralement de 0,01 % à 1 %, et de préférence de 0,02 % à 0,5 % en poids, par rapport au poids total de la composition.

On décrit plus en détail ci-après diverses classes de silicones qui peuvent être utilisées dans la composition de l'invention.

Les silicones , utilisées conformément à l'invention sont des polyorganosiloxanes , pouvant se présenter sous forme d'huiles, de gommes ou de résines.

Les polyorganosiloxanes plus particulièrement utilisés , conformément à l'invention , sont choisis parmi les **silicones volatiles** ,celles-ci possédant un point d'ébullition généralement compris entre 60 et 260°C , ou bien des **silicones non volatiles** choisies en particulier parmi les polyalkylsiloxanes, les polyarylsiloxanes, les polyalkylarylsiloxanes, les copolymères polyéthersiloxanes organomodifiés ou non ou des gommes et résines de silicones, des polysiloxanes modifiés par des groupements organofonctionnels ainsi que leurs mélanges.

Ils sont choisis plus particulièrement parmi les polyalkylsiloxanes, parmi lesquels on peut citer principalement les polydiméthylsiloxanes linéaires à groupements terminaux triméthylsilyle ayant une viscosité de $5.10^{-6}$ à 2,5 $m^2/s$ à 25°C et de préférence $1.10^{-5}$ à 1 $m^2/s$.

Parmi ces polyalkylsiloxanes , on peut citer , à titre non limitatif ,les produits commerciaux suivants :

. les huiles SILBIONE des séries 47 et 70 047 commercialisées par RHONE POULENC telles que par exemple l'huile 47 V 500.000

. les huiles de la série 200 de DOW CORNING,

. les huiles VISCASIL de GENERAL ELECTRIC et certaines huiles des séries SF (SF 96 , SF 18) de GENERAL ELECTRIC,

On peut également citer les polydiméthylsiloxanes linéaires à groupements terminaux diméthylsilanol tels que les huiles de la série 48 de RHONE POULENC.

Dans cette classe de polyalkysiloxanes , on peut également citer les produits vendus sous les dénominations ABIL WAX 9800 et 9801 par la société GOLDSSCHMIDT qui sont des polyalkyl($C_1$-$C_{20}$)siloxanes.

Parmi les polyalkylarylsiloxanes, on peut citer les polydiméthyl méthylphénylsiloxanes, polydiméthyldiphénylsiloxanes linéaires et/ou ramifiés de viscosité de $1.10^{-5}$ à $5.10^{-2}$ $m^2/s$ à 25°C.

Parmi ces polyalkylarylsiloxanes , on peut citer par exemple et sans limitation les produits commerciaux suivants:

. les huiles SILBIONE de la série 70 641 de RHONE POULENC,
. les huiles des séries RHODORSIL 70 633 et 763 de RHONE POULENC
. l'huile DC 556 Cosmetic Grad Fluid de DOW CORNING,
. les silicones de la série PK de BAYER comme le produit PK20 ,
. les silicones des séries PN, PH de BAYER comme les produits PN 1000 et PH 1000,
. certaines huiles des séries SF de GENERAL ELETRIC telles que SF 1023 , SF 1154 , SF 1250 , SF 1265.

**Les gommes de silicone** utilisables , conformément à la présente invention , sont des polydiorganosiloxanes ayant des masses moléculaires élevées , comprises entre 200.000 et 1.000.000 , utilisés seuls ou en mélange dans un solvant. Ce solvant peut être choisi parmi les silicones volatiles , les huiles polydiméthylsiloxanes (PDMS) , les huiles polyphénylméthylsiloxanes (PPMS) , les isoparaffines , le chlorure de méthylène , le pentane , le dodécane , le tridécane , le tétradécane ou leurs mélanges.

On peut citer plus particulièrement les produits suivants:

les gommes polydiméthylsiloxane/méthylvinylsiloxane,
polydiméthylsiloxane/diphénylsiloxane,
polydiméthylsiloxane/phénylméthylsiloxane,
polydiméthylsiloxane/diphénylsiloxane/méthylvinylsiloxane.

Les produits plus particulièrement utilisables sont des mélanges tels que :

. les mélanges formés à partir d'un polydiméthylsiloxane hydroxylé en bout de chaîne (dénommé dimethiconol selon la nomenclature du dictionnaire CTFA) et d'un polydiméthylsiloxane cyclique (dénommé cyclomethicone selon la nomenclature du dictionnaire CTFA) tel que le produit Q2 1401 vendu par la société DOW CORNING,
. les mélanges formés à partir d'une gomme polydiméthylsiloxane avec une silicone cyclique tel que le produit SF 1214 Silicone Fluid de GENERAL ELECTRIC.( qui est une gomme SE 30 , correspondant à une diméthicone , ayant un poids moléculaire de 500 000 solubilisée dans la SF 1202 Silicone Fluid (correspondant au décaméthylcyclopentasiloxane)
. les mélanges de deux PDMS de viscosités différentes , notamment d'une gomme PDMS et d'une huile PDMS , tels que le produit SF 1236 de la société GENERAL ELECTRIC . Le produit SF 1236 est le mélange d'une gomme SE 30 définie ci-dessus d'une viscosité de 20 $m^2/s$ et d'une huile SF 96 d'une viscosité de $5.10^{-6}$ $m^2/s$ ( 15% de gomme SE 30 et 85% d'huile SF 96).

**Les résines d'organopolysiloxanes** , conformes à la invention sont des systèmes siloxaniques réticulés renfermant les unités $R_2SiO_{2/2}$ , $RSiO_{3/2}$ et $SiO_{4/2}$.

Parmi ces composés , les produits plus particulièrement préférés sont ceux dans lesquels R désigne un alkyle inférieure ou un phényle.

Parmi ces résines , on peut citer le produit vendu sous la dénomination DOW CORNING 593 ou ceux vendus sous les dénominations Silicones Fluid SS 4230 et SS 4267 par la société GENERAL ELECTRIC et qui sont de type diméthyl/ triméthyl siloxane.

**Les silicones organomodifiées** sont des silicones définies ci-dessus et comportant dans leur structure un ou plusieurs groupements organofonctionnels directement fixés sur la chaîne siloxanique ou fixés par l'intermédiaire d'un radical hydrocarboné.

Parmi ces silicones , on peut citer , par exemple , les silicones comportant :

**1-** des groupements **polyéthylèneoxy et/ou polypropylèneoxy** comportant éventuellement des groupes alkyles tels que :

. le produit dénommé diméthicone copolyol vendu par la société DOW CORNING sous la dénomination DC 1248 ,et l'alkyl($C_{12}$) méthicone copolyol vendue par la société DOW CORNING sous la dénomination Q2 5200
. les huiles SILWET L 722 , L 7500 , L 77 , L 711 de la société UNION CARBIDE.

**2-** des groupements **aminés substitués ou non** comme les produits vendus sous la dénomination GP4 Silicone Fluid et GP 7100 par la société GENESEE ou les produits vendus sous les dénominations Q2 8220 et DC 929 par la société DOW CORNING. Les groupements aminés substitués sont en particulier des groupements aminoalkyle($C_1$-$C_4$),

**3-** des groupements **thiols** comme dans les GP 72 A et GP 71 de GENESEE ,
**4-** des groupements **carboxylates** comme dans les produits décrits dans le brevet EP 186 507 de la société CHISSO CORPORATION ,
**5-** des groupements **alkoxylés** , comme le produit vendu sous la dénomination Silicone copolymer F-755 par SWS SILICONES , et ABIL WAX 2428 ,2434 et 2440 par la société GOLDSCHMIDT,
**6-** des groupements **hydroxylés** , comme les polyorganosiloxanes à fonction hydroxyalkyle , décrits dans la demande de brevet français N° FR-85 163 34 , répondant à la formule suivante (II):

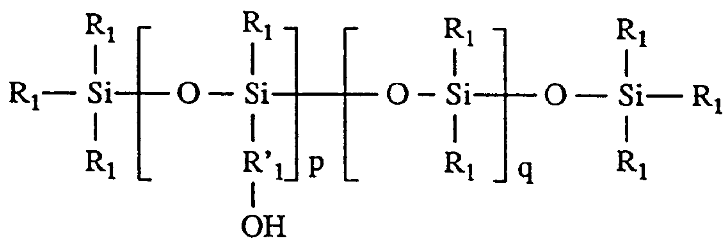

( I I )

dans laquelle:

- les radicaux $R_1$ , identiques ou différents , sont choisis parmi les radicaux méthyle et phényle , au moins 60% en mole des radicaux $R_1$ désignant méthyle ;
- le radical $R'_1$ est un chainon alkylène divalent hydrocarboné en $C_2$-$C_{18}$ ;
- p est compris entre 1 et 30 inclus ;
- q est compris entre 1 et 150 inclus ;

**7-** des groupements **acyloxyalkyle** , comme par exemple les polyorganosiloxanes décrits dans la demande de brevet français N° FR-88 17 433 , répondant à la formule suivante (III):

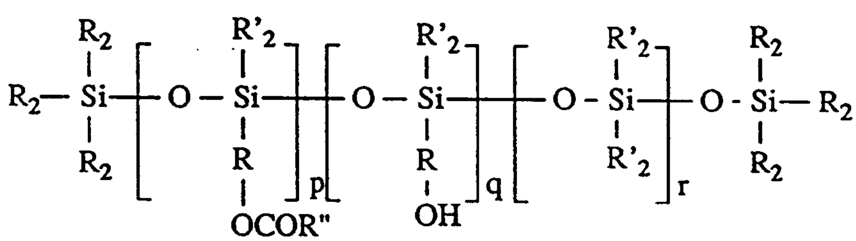

(III)

dans laquelle:

- $R_2$ désigne méthyle , phényle , -OCOR", hydroxyle , un seul des radicaux $R_2$ par atome de silicium peut être OH ;
- $R'_2$ désigne méthyle , phényle , au moins 60% molaire de l'ensemble des radicaux $R_2$ et $R'_2$ désignant méthyle ;
- R" désigne alkyle ou alkényle en $C_8$-$C_{20}$ ;
- R désigne un radical alkylène hydrocarboné divalent, linéaire ou ramifié, en $C_2$-$C_{18}$ ;
- r est compris entre 1 et 120 inclus ;
- p est compris entre 1 et 30 ;
- q est égal à 0 ou est inférieure à 0,5p , p+q étant compris entre 1 et 30 ; les polyorganosiloxanes de formule (III) peuvent contenir des groupements

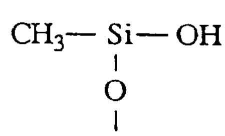

dans des proportions ne dépassant pas 15% de la somme p+q+r.

Les composés de formule (III) peuvent être préparés par estérification de polyorganosiloxanes à fonction hydroxyalkyle de formule (II) ci-dessus.

L'estérification s'effectue de façon connue , avec un acide R"COOH ou l'anhydride d'acide , à une température comprise entre 100 et 250°C en présence éventuellement d'un catalyseur comme le chlorure d'aluminium ou le chlorure de zinc ou d'un acide fort comme l'acide chlorhydrique ou l'acide sulfurique.

On peut également effectuer une transestérification par chauffage à 100-150°C d'un ester méthylique de formule $R"COOCH_3$ et d'un diorganopolysiloxane de formule (II) , en présence d'un catalyseur acide comme l'acide paratoluènesulfonique ou une terre acide de type Montmorillonite ( KATALYSATOR KSF/O vendu par la société SUD-CHEMIE - A.G.MUNCHEN ).

**8-** des groupements **anioniques** de type

- **carboxylique** tels que les groupements alkylcarboxyliques comme dans le produit X-22-3701E de la société SHIN-ETSU
- **2-hydroxy alkylsulfonate**
- **2-hydroxyalkylthiosulfate** tels que dans les produits vendus par la société GOLDSCHMIDT sous les dénominations ABIL S201 et ABIL S 255 .

**Les silicones volatiles** sont plus particulièrement choisies parmi :

**1** - les silicones cycliques comportant de 3 à 7 atomes de silicium et de préférence 4 à 5 atomes de silicium telles que plus particulièrement l'octaméthylcyclotétrasiloxane vendu sous la dénomination Volatile Silicone 7207 par UNION CARBIDE ou Silbione 70045 V 2 par RHONE POULENC ,ou le décaméthylcyclopentasiloxane vendu en particulier sous la dénomination Volatile Silicone 7158 par la société UNION CARBIDE ,ou bien Silbione 70045 V 5 vendu par la société RHONE POULENC ainsi que leurs mélanges.

On peut citer également les cyclocopolymères tel que le diméthylsiloxane / méthylalkylsiloxane , et notamment la silicone volatile FZ 3109 vendue par la société UNION CARBIDE , présentant la structure :

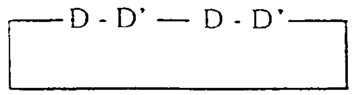

avec D : 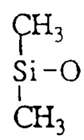 D' : 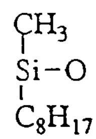

On peut également citer les mélanges de silicones cycliques avec des composés dérivés du silicium tels que le mélange d'octaméthylcyclotétrasiloxane et de tétra triméthylsilyl pentaérythritol ( 50/50 ) et le mélange d'octaméthylcyclotétrasiloxane et d'oxy-1,1'(hexa-2,2,2',2',3,3'triméthylsilyloxy)bis-néopen tane

**2** - les silicones volatiles linéaires ayant 2 à 9 atomes de silicium et possédant une viscosité inférieure ou égale à $5.10^{-6}$ $m^2/s$ à 25°C. Des silicones de ce type sont en particulier constituées par l'hexaméthyldisiloxane vendu sous la dénomination Silbione 70 041 V 0,65 par la société RHONE POULENC et par le décaméthyltétrasiloxane vendu sous la dénomination SH 200 par la société TORAY SILICONE . Des silicones entrant dans cette classe sont également décrits dans l'article publié dans Cosmetics and toiletries , Vol. 91 , Jan 76 , p. 27-32 . - TODD & BYERS "Volatile Silicone fluids for cosmetics".

Les polyorganosiloxanes plus particulièrement préférés, conformément à l'invention, sont :

- les silicones volatiles telle que la décaméthyl cyclopentasiloxane vendue sous la dénomination huile silbione 70045V5 par la Société RHONE POULENC.
- les silicones non volatiles choisies dans la famille des polyalkylsiloxanes linéaires à groupements terminaux triméthylsilyl telles que les huiles, de viscosité comprise entre $5.10^{-5}$ et $5.10^{-2}$ $m^2/s$ à 25 ° C, des séries 70047 et 47 et plus particulièrement l'huile 70047V5000 commercialisée par RHONE POULENC, ou les polyalkylarylsiloxanes telle que l'huile ABIL AV 1000 vendue par la Société GOLDSCHMIDT.

Les polymères filmogènes utilisables dans la composition de l'invention peuvent être des polymères anioniques, cationiques, non ioniques ou amphotères. Ces polymères filmogènes, de même que leur utilisation dans des compositions cosmétiques pour cheveux, sont connus.

On décrit plus en détail ci-après certains polymères filmogènes utilisables dans les compositions de l'invention.

Les polymères cationiques utilisés conformément à l'invention sont des polymères du type polyamine, polyaminopolyamide ou polyammonium quaternaire dans lesquels le groupement amine ou ammonium fait partie de la chaîne polymère ou est reliée à celle-ci et ils ont un poids moléculaire compris entre 500 et 3.000.000.

Les polymères cationiques utilisables conformément à l'invention sont choisis notamment parmi les polymères suivants :

1) les copolymères vinyl-pyrrolidone-acrylate ou méthacrylate de dialkylaminoalkyle (quaternisés ou non), tels que ceux vendus sous les dénominations Gafquat par la Gaf Corp. comme par exemple le "copolymère 845", le "Gafquat 734 ou 735" décqrits notamment plus en détail dans le brevet français 2.077.141 et le brevet français 2.393.573,

2) les dérivés d'éthers de cellulose comportant des groupements ammonium quaternaires tels que ceux décrits dans le brevet français 1.492.597 et notamment les polymères vendus sous les dénominations JR tels que JR 125, JR 400, JR 30 M et LR, tels que LR 400 et LR 30 M par la Société Union Carbide Corp. les dérivés de cellulose cationiques tels que les CELQUAT L 200 et CELQUAT H 100 vendus par la Société National Starch et décrits dans le brevet américain 4.131.576,

3) les polysaccharides cationiques comme décrits dans les brevets américains 3.589.978 et 4.031.307 et en particulier le Jaquar C.13 S vendu par la Société Meyhall,

4) les polymères cationiques choisis dans le groupe formé par:

a) les polymères contenant des motifs de formule :
- A - Z - A -Z - (I) dans laquelle A designe un radical comportant deux fonctions amine et de préference pipérazinyle et Z désigne le symbole B ou B'; B et B' identiques ou différents désignent un radical bivalent qui est un radical alkylène à chaîne droite ou ramifiée, comportant jusqu'à 7 atomes de carbone consécutifs dans la chaîne principale, non substitué ou substitué par des groupements hydroxyle et pouvant comporter en oure des atomes d'oxygène, d'azote, de soufre, 1 à 3 cycles aromatiques et/ou hétérocycles; les atomes d'oxygène, d'azote et de soufre étant présents sous forme de groupements éther ou thioéther, sulfoxyde, sulfone, sulfonium, amine, alkylamine, alkénylamine, benzylamine, oxyde d'amine, ammonium quaternaire, amide, imide, alcool, ester et/ou uréthane; ces polymères et leur procédé de préparation sont décrits dans le brevet français 2.162.025,

b) les polymères contenant des motifs de formule : - A - $Z_1$ - A - $Z_1$ - (II) dans laquelle A desige un radical comportant deux fonctions amine et de préférence pipérazinyle et $Z_1$ désigne le symbole $B_1$ ou $B'_1$ et il signifie au moins une fois le symbole $B'_1$; $B_1$ désigne un radical bivalent qui est un radical alkylène ou hydroxyalkylène à chaîne droite ou ramifiée ayant jusqu'à 7 atomes de carbone consécutifs dans la chaîne principale, $B'_1$ est un radical bivalent qui est un radical alkylène à chaîne droite ou ramifiée ayant jusqu'à 7 atomes de carbone consécutifs dans la chaîne principale, non substitué ou substitué par un ou plusieurs radicaux hydroxyle et interrompu par un ou plusieurs atomes d'azote, l'atome d'azote étant substitué par une chaîne alkyle interrompue éventuellement par un atome d'oxygène et comportant éventuellement une ou plusieurs fonctions hydroxyle; ces polymères et leur procédé de préparation sont décrits dans le brevet français 2.280.361.

c) les produits d'alcoylation avec les halogénures d'alcoyle ou de benzyle, tosylates ou mésylates d'alcoyle inférieur et les produits d'oxydation des polymères de formules (I) et (II) ci-dessus indiquée sous a) et b).

5) Les cyclopolymères ayant un poids moléculaire de 20.000 à 3.000.000 tels que les homopolymères comportant comme constituant principal de la chaîne, des motifs répondant à la formule (III) ou (III').

(III) 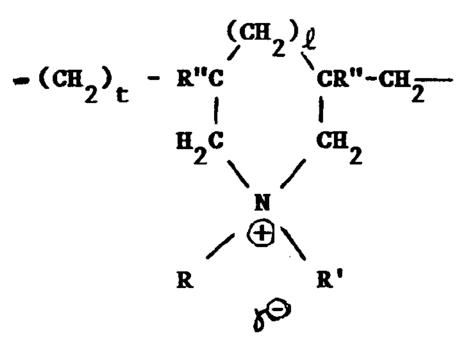

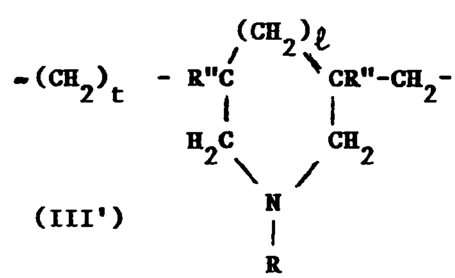 (III')

dans laquelle l et t sont égaux à 0 ou 1, et la somme l + t = 1, R" désigne hydrogène ou méthyle, R et R' désignent indépendamment l'un de l'autre, un groupement alcoyle ayant de 1 à 22 atomes de carbone, un groupement hydroxyalcoyle dans lequel le groupement alcoyle a de préférence 1 à 5 atomes de carbone, un groupement amidoalcoyle inférieur et où R et R' peuvent désigner conjointement avec l'atome d'azote auquel ils sont rattachés des groupements hétérocycliques tels que pipéridinyle ou morpholinyle, ainsi que les copolymères comportant des unités de formule III ou III' et des unités dérivées d'acrylamide ou de diacétone acrylamide, $Y^{\ominus}$est un anion tel que bromure,chlorure, acétate, borate, citrate, tartrate, bisulfate, bisulfite, sulfate,

phosphate.

Parmi les polymères d'ammonium quaternaire du type ci-dessus défini, on citera l'homopolymère de chlorure de diméthyl diallyl ammonium vendu sous la dénomination MERQUAT 100 ayant un poids moléculaire inférieur à 100.000 et le copolymère de chlorure de diméthyl diallyl ammonium et d'acrylamide ayant un poids moléculaire supérieur à 500.000 et vendu sous la dénomination de MERQUAT 550 par la Société MERCK.

Ces polymères sont décrits dans le brevet français 2.080.759 et son certificat d'addition n°2.190.406.

Les polymères anioniques sont des polymères ayant un poids moléculaire compris entre 500 et 3.000.000 et comportant des groupes carboxyliques et/ou sulfoniques.

Les groupements carboxyliques sont apportés dans les polymères anioniques par des mono ou diacides carboxyliques insaturés représentés notamment par la formule :

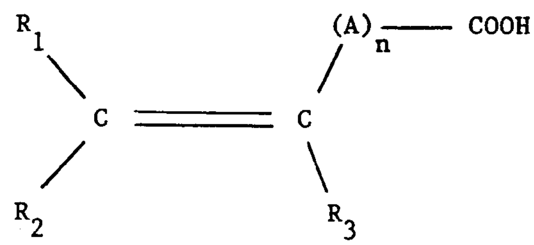

dans laquelle n est un nombre entier de 0 à 10, A désigne un groupement méthylène éventuellement relié à l'atome de carbone du groupement insaturé ou au groupement méthylène voisin lorsque n est supérieur à 1 par l'intermédiaire d'un hétéroatome tel que oxygène, soufre, $R_1$ désigne un atome d'hydrogène, un groupement phényle, benzyle, $R_2$ désigne un atome d'hydrogène, un groupement alcoyle inférieur, carboxyle, $R_3$ désigne un atome d'hydrogène, un groupement alcoyle inférieur, $-CH_2-COOH$, phényle, benzyle.

Dans la formule précitée un radical alcoyle inférieur désigne de préférence un groupement ayant 1 à 4 atomes de carbone et en particulier méthyle, éthyle....,

Les polymères anioniques préférés utilisés conformément à l'invention sont choisis notamment parmi :

- les homo- ou copolymère d'acides acrylique ou méthacrylique ou leurs sels et en particulier les produits vendus sous les dénominations VERSICOL F. ou K. par la Société ALLIED COLLOID, ULTRAHOLD 8 par la Société CIBA GEIGY, les copolymères d'acide acrylique et d'acrylamide vendus sous la forme de leur sel de sodium sous les dénominations RETEN 421, 423 ou 425 par la Société HERCULES, le polyméthacrylate de sodium vendu sous la dénomination DARVAN n°7 par la Société Van der Bilt, les sels de sodium d'acides polyhydroxycarboxyliques vendus sous la dénomination HYDAGEN F par la Société HENKEL;
- les copolymères dérivés d'acide crotonique tels que ceux comportant dans leur chaîne des motifs acétate ou propionate de vinyle et éventuellement d'autres monomères tels que esters allylique ou méthallylique, éther vinylique, ou ester vinylique d'un acide carboxylique saturé à longue chaîne hydrocarbonés tels que ceux comportant au moins 5 atomes de carbone ou encore un ester vinylique, allylique ou méthallylique d'un acide carboxylique ou cyclique, ces polymères pouvant éventuellement être greffés et réticulés. De tels polymères sont décrits entre autres dans les brevets français 1.222.944, 1.580.545, 2.265.782, 2.265.781, 1.564.110 et.2.439.798. Des produits commerciaux entrant dans cette classe sont les résines 28-29-30, 26-13-14 et 28-13-10 vendues par la Société National Starch.
- Les copolymères des acides acrylique ou méthacrylique avec les esters d'acides acrylique ou méthacrylique,
- les copolymères d'acide crotonique et d'esters vinyliques greffés sur un polyalcoylèneglycol tel que le polyéthylèneglycol comme le copolymère acide crotonique/acétate de vinyle greffé sur du polyéthylèneglycol vendu sous la dénomination ARISTOFLEX A par la Société HOECHST.

Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters; ces polymères peuvent être estérifiés. De tels polymères sont décrits en particulier dans les brevets des E.U.A. 2.047.398, 2.723.248, 2.102.113, le brevet britannique 839.805. On peut citer notamment les polymères vendus sous les dénominations GANTREZ AN, S ou ES par la Société Général Anilin ou EMA 1325 ou 91 par la Société MONSANTO. Des polymères entrant également dans cette classe sont les copolymères d'anhydride maléique, citraconique, itaconique et d'un ester allylique ou méthallylique comportant éventuellement un groupement acrylamide ou méthacrylamide dans leur chaîne, monoestérifiés ou monoamidifiés décrits dans les brevets français 2.350.834 et 2.357.241 de la demanderesse.

Les polymères à groupement sulfonique utilisables conformément à l'invention sont choisis notamment

parmi :

- les sels de l'acide polystyrène sulfonique tels que les sels de sodium vendus sous la dénomination Flexan 500 ayant un poids moléculaire d'environ 500.000 ou sous la dénomination Flexan 130 ayant un poids moléculaire d'environ 100.000 par la Société National STARCH. De tels composés sont décrits notamment dans le brevet français 2.198.719.
- les sels de polyacrylamide sulfoniques, tels que ceux mentionnés dans le brevet américain 4.128.631 et plus particulièrement l'acide polyacrylamidoéthylpropane sulfonique vendu sous la dénomination COSMEDIA POLYMER HSP 1180 par la Société HENKEL.

Les polymères filmogènes amphotères utilisables selon l'invention sont des polymères comportant des motifs A et B répartis statistiquement dans la chaîne polymère où A désigne un motif dérivant d'un monomère comportant au moins un atome d'azote basique et B désigne un motif dérivant d'un monomère acide comportant un ou plusieurs groupements carboxyliques ou sulfoniques ou bien A et B peuvent désigner des groupements dérivant de monomères zwittérioniques de carboxybétaïne;

A et B peuvent également désigner une chaîne polymère cationique comportant des groupements amine secondaire, tertiaire ou quaternaire, dans laquelle au moins l'un des groupements amine porte un groupement carboxylique ou sulfonique relié par l'intermédiaire d'un radical hydrocarboné ou bien A et B font partie d'une chaîne d'un polymère à motif éthylène alpha, béta-dicarboxylique dont l'un des groupements carboxylique a été amené à réagir avec une polyamine comportant un ou plusieurs groupements amine primaire ou secondaire.

Les polymères amphotères répondant à la définition indiquée ci-dessus plus particulièrement préférés sont choisis parmi les polymères suivants :

1) Les polymères comportant des motifs dérivant

a) d'au moins un monomère choisi parmi les acrylamides ou les méthacrylamides substitués à l'azote par un radical alkyle,

b) d'au moins un comonomère acide contenant un ou plusieurs groupements carboxyliques réactifs, et

c) au moins un comonomère basique tel que des esters à substituants amines primaire, secondaire, tertiaire et quaternaire des acides acrylique et méthacrylique et le produit de quaternisation du méthacrylates de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertio-octyl acrylamide, le N-octylacrylamide, le N-décylacrylamide, le N-dodécylacrylamide ainsi que les méthacrylamides correspondants. Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone de l'acide maléïque ou de l'acide fumarique.

Les comonomères basiques préférés sont des méthacrylates d'amino-éthyle, de butyl aminoéthyle, de N,N'-diméthylaminoéthyle, de N-tertio-butylaminoéthyle.

Comme composé représentatif de cette classe on peut citer l'Amphomer qui est un copolymère N-tert octyl acrylamide/méthacrylate de méthyle/méthacrylate d'hydroxypropyl/acide acrylique/méthacrylate de tert-butyl aminoéthyle vendu par la Société National Starch.

2) Les polymères dérivés du chitosane comportant des motifs monomères répondant aux formules suivantes :

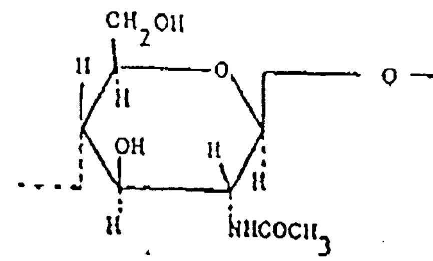

(A)

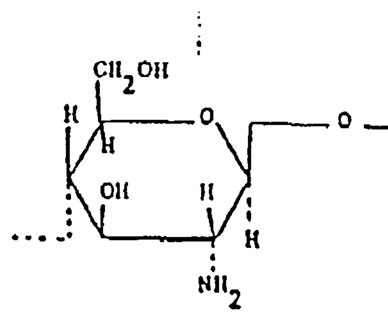

(B)

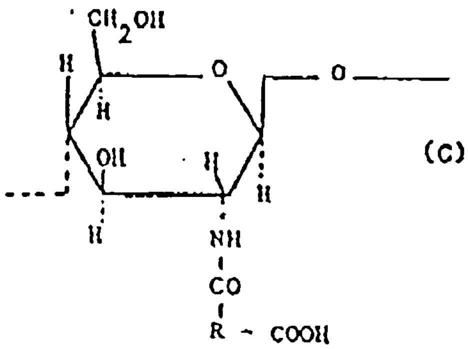

(C)

dans lesquelles le motif A est présent dans des proportions comprises entre 0 et 30%, B est présent dans des proportions comprises entre 5 et 50% et C est présent dans des proportions comprises entre 30 et 90%. Dans la formule C, R représente un radical de formule :

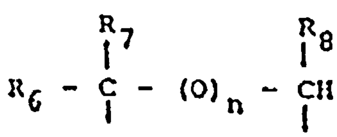

dans laquelle si n = 0, $R_6$, $R_7$ et $R_8$ identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux $R_6$, $R_7$ et $R_8$ étant dans ce cas un atome d'hydrogène; ou n est égal à 1, auquel cas $R_6$, $R_7$ et $R_8$ représentent chacun un atome d'hydrogène, ainsi que les sels formés par ces composés avec des bases ou des acides.

Les polymères dérivés du chitosane, utilisables dans les compositions selon l'invention peuvent être préparés par acylation du chitosane avec un anhydride d'acide conformément au mode opératoire décrit dans l'exemple 1 du brevet français 2 137 684 ou dans le brevet US 3 879 376. Un polymère dérivé du chitosane, plus particulièrement préféré selon l'invention, comporte de 0 à 20% en poids de motifs (A), de 40 à 50% en poids de motifs (B) et de 40 à 50% en poids de motifs (Cl), ce dernier motif ayant la formule :

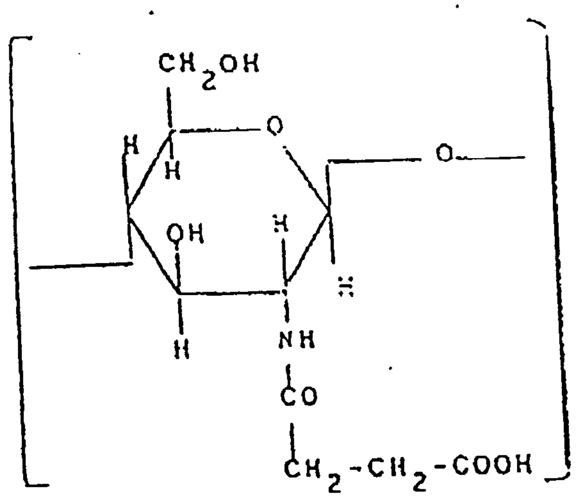

$(C_1)$

Ce polymère préféré constitué de motifs (A), (B) et $(C_1)$ sera dénommé par la suite : polymère $(P_1)$.

3) Des polymères amphotères du type -A-Z-A-Z- choisis parmi a) les polymères obtenus par action de l'acide chloracétique ou le chloracétate de sodium sur les composés comportant au moins un motif de formule

-A-Z-A-Z-A- (V)

ou A désigne un radical

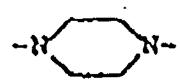

dans laquelle % désigne la symbole B ou B', B ou B' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques: les atomes d'oxygène d'azote et de soufre étant présents sous forme de groupement éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthane.

b) Les polymères obtenus par action de l'acide chlorocétique ou du chlorocétate de sodium sur les composés de formule -A-Z-A-Z- (V) où A désigne un radical

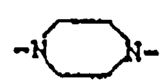

où Z désigne B ou B' et au moins une fois B'; B ayant la signification indiquée ci-dessus et B' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions hydroxyle et/ou carboxyle ainsi que les sels d'ammonium quaternaires résultant de la réaction de l'acide chloracétique ou du chloracétate de soude sur les polymères (V).

4) Les kératines sulfoniques sont des kératines d'un poids moléculaire compris entre 10.000 et 100.000, obtenues à partir de plumes d'oie ou de poulet ou plus avantageusement encore de sabots ou de cornes.

Cette kératine est obtenue par oxydation de tout ou partie des liaisons disulfures des groupements cystine de la kératine en groupements acide cystéique, cette oxydation étant suivie ou non d'une salification des groupements acides-$SO_3H$, l'oxydation étant avantageusement effectuée en milieu acide, tel que l'acide formique, au moyen d'un agent oxydant, tel que l'eau oxygénée.

5) Les copolymères de diallyl dialkyl ($C_1$-$C_4$) ammonium/acide acrylique comme le produit vendu sous la dénomination MERQUAT 280 par la Société MERCK et qui est un copolymère de chlorure de diallyle diméthyl ammonium/acide acrylique.

Les polymères non ioniques utilisables selon l'invention sont notamment :

- la polyvinylpyrrolidone ou les copolymères de vinylpyrrolidone avec des comonomères non ioniques tel que le copolymère polyvinylpyrrolidone/acétate de vinyle vendu sous la dénomination PVP/VA S630 par la Société GAF.
- les homopolymères ou copolymères vinylique non ioniques tel que l'alcool polyvinylique vendu sous la dénomination MOWIOL 4088 par la Société HOECHST.
- les poly-β-alanines décrites plus particulièrement dans la demande de brevet belge n°208.516 déposée par la demanderesse.

Ces polymères comportent de 50 à 100% de motifs répétitifs de formule :

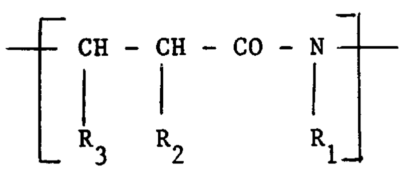

(I)

et de 0 à 50% de motifs répétitifs du type polyacrylamide correspondant à la formule suivante :

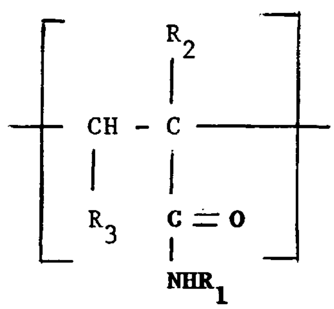

(II)

dans lesquelles $R_1$ représente un atome d'hydrogène, ou un radical pris dans le groupe constitué par les radicaux suivants

(i)

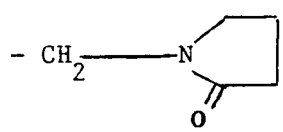

(ii) - $CH_2OH$

(iii) - $CH_2)_{n'}$ - $CH_3$, n' étant O, un nombre entier de 1 à 11, et

(iv)

$$-(CH_2-CH_2-O)_m-H,$$

m étant compris entre 1 et 10,

et $R_2$ et $R_3$ représentent un atome d'hydrogène ou un radical méthyle.

Ces polymères sont préparés plus particulièrement par polymérisation de l'acrylamide, comme décrit dans le brevet américain 4.082.730. Ces polymères ont de préférence un poids moléculaire compris entre 500 et 100.000 et plus particulièrement entre 2.000 et 60.000.

Les dérivés d'acide polyaspartique tels que ceux décrits dans le brevet français n°77.27769 (n° de publication : 2.403.076).

Lesdits dérivés d'acide polyaspartique répondent à la formule III :

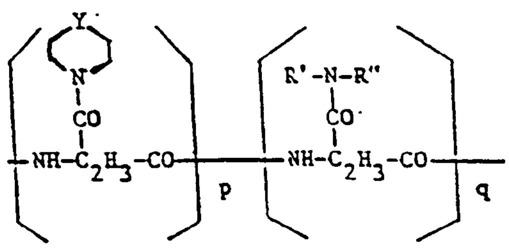

dans laquelle :

R' représente un atome d'hydrogène, un groupement hydroxyalcoyle inférieur, un groupement hydroxyalcoyloxyalcoyle inférieur, un groupement alcoyle ayant au plus 18 atomes de carbone ou un groupement alcényle ayant 2 à 18 atomes de carbone;

R" représente un atome d'hydrogène, un groupement hydroxyalcoyle inférieur ou un groupement alcoyle inférieur;

Y représente méthylène, -O- , -N(R"')- ou

$$-\underset{\oplus}{N}(R''')(R'''')-.Z^{\ominus}$$

R"' et R"" représentant -H, alkyle 1-18C, alcényle 2-18C, et $Z^{\ominus}$ étant un anion.

Les polymères filmogènes particulièrement préférés sont :

Pour les polymères cationiques :

- les copolymères vinylpyrrolidone/acrylate ou méthacrylate de diaminoalkyle du groupe 1 tel que le produit vendu sous la dénomination GAFQUAT 734 par la Société GAF.
- les dérivés d'éthers de cellulose cationiques et les dérivés de cellulose cationiques du groupe 2 et notamment les produits vendus sous les dénominations JR 400 et CELQUAT LOR,
- les cyclopolymères du groupe 9 et notamment le produit vendu sous la dénomination MERQUAT 550.

Pour les polymères anioniques :

- les polymères dérivés d'acide ou d'anhydride maléique tel que le polymère vendu sous la dénomination GANTREZ ES 425.

Pour les polymères amphotères :

- les polymères dérivés du chitosane du groupe 2 tel que le polymère dénomé ci-dessus ($P_I$)
- les kératines sulfoniques.

Pour les polymères non ioniques :

- le copolymère vinylpyrrolidone/acétate de vinyle tel que le produit vendu sous la dénomination PVP/VA S 630 par la Société GAF.

Les polymères filmogènes sont présents, dans les compositions de l'invention en proportion pondérale au moins égale à 0,1 % et inférieure à 2 %, et en particulier entre 0,1 et 1 %.

De préférence, le rapport pondéral polymère filmogène/silicone est supérieur à 0,25 et inférieur à 8, et en particulier supérieur à 0,5 et inférieure à 6.

Les compositions cosmétiques de l'invention peuvent aussi contenir un ou plusieurs ingrédients secondaires usuels tels que des agents épaississants, des agents stabilisants, des parfums ou des agents conservateurs.

Les compositions sans agent épaississant sont des lotions fluides. Les compositions avec agent épaississant sont des lotions ou des gels fluides.

Les agents épaississants sont plus particulièrement choisis parmi les acides polyacryliques réticulés par un agent polyfonctionnel tels que les produits vendus sous la dénomination CARBOPOL par la Société Goodrich, comme les Carbopols 910, 934, 934P, 940, 941, 1342, ou des dérivés cellulosiques comme l'hydroxyméthylcellulose, la carboxyméthylcellulose, l'hydroxybutylcellulose, l'hydroxypropylcellulose, et plus particulièrement l'hydroxyéthylcellulose, tels que les produits vendus sur la dénomination "NATROSOL" (150, 250) par la société HERCULES ou "CELLOSIZE" (QP et WP) par la société UNION CARBIDE, la méthylhydroxypropyl cellulose, en particulier les produits vendus sous la dénomination "METHOCEL" (E, F, J, K) par la société

DOW CHEMICAL ou des hétérobio-polysaccharides tels que par exemple les gommes de xanthane commercialisées sous les marques "KELTROL" et "KELZAN" par la société KELCO, "RHODOPOL" et "RHODIGEL" par la société RHONE POULENC, ou "ACTIGUM" par la société CECA/SATIA.

Lorsqu'on utilise des épaississants, ceux-ci sont de préférence choisis parmi les Carbopols et utilisés de préférence à une concentration telle que la viscosité de la composition soit au plus égale à 25 poises (soit 2,5 Pa.s) environ à 25°C (viscosimètre Contraves; corps de mesure n°3; temps de rotation 10 minutes, à 200 tours/min.).

Comme agents stabilisants, on peut citer les esters phosphoriques d'alcools gras. Ils sont utilisés généralement à une concentration inférieure à 1%.

Les agents conservateurs sont par exemple l'acide parahydroxybenzoïque, ses sels et esters, l'acide sorbique et ses sels, la diméthyloldiméthylhydantoïne et les dérivés d'imidazolidinyl urée. Ils sont utilisés aux concentrations efficaces usuelles.

Le pH des compositions obtenues selon l'invention peut varier de 3 à 10. Le pH peut éventuellement être ajusté à l'aide d'un agent modificateur de pH usuel.

Les compositions selon l'invention sont obtenues par formation à chaud d'une microémulsion. Plus précisément, ces compositions sont obtenues par un procédé principalement caractérisé par le fait que l'on chauffe la cire et l'émulsionnant à une température supérieure à la température de fusion de la cire et non supérieure à 100°C, éventuellement en présence d'une partie de l'eau, jusqu'à fusion complète de la cire, que l'on ajoute progressivement l'eau, ou le restant de l'eau, portée à une température au moins égale à ladite température, en agitant, jusqu'à formation d'une microémulsion de cire dans une phase continue aqueuse, puis que l'on laisse refroidir jusqu'à la température ambiante. On obtient une microdispersion stable de cire.

Les ingrédients liposolubles, par exemple des céramides, sont généralement ajoutés à la cire, avant la réalisation de la microdispersion.

Les ingrédients hydrosolubles peuvent être ajoutés dans l'eau utilisée pour réaliser la microdispersion, ou dans la microdispersion de cire finalement obtenue.

De même, les ingrédients secondaires éventuellement présents dans la composition sont ajoutés selon les cas soit dans les produits de départ, soit dans la composition terminée.

Selon un mode d'exécution particulier, on ajoute le polymère filmogène et la silicone, sous agitation, après obtention de la microdispersion de cire.

Les compositions de l'invention sont diluables à l'eau sans nuire à la stabilité de la microdispersion. Elles peuvent donc se présenter sous la forme de compositions concentrées dont on peut ajuster la proportion des ingrédients à une valeur désirée par simple addition d'eau.

Les compositions de l'invention peuvent être appliquées sur cheveux secs ou mouillés, propres ou non. Elles peuvent être rincées ou non rincées.

Malgré la présence d'une cire dans la composition, aucun aspect gras n'est communiqué aux cheveux, même en l'absence de rinçage. En outre, malgré la présence d'une forte proportion d'eau dans la composition, le séchage ne pose pas de problèmes et s'effectue rapidement.

L'invention a également pour objet un procédé de traitement cosmétique des cheveux et/ou du cuir chevelu, caractérisé par le fait que l'on applique sur les cheveux une quantité efficace d'une composition telle que définie précédemment.

La fréquence d'application peut varier par exemple entre une application quotidienne et une application hébdomadaire.

Les exemples suivants illustrent l'invention sans toutefois la limiter.

Les noms commerciaux utilisés dans les exemples représentent les produits suivants :

**GANTREZ ES 425** : Copolymère méthylvinyléther/anhydride maléique monoestérifié au butanol à 50% de matière active dans l'éthanol vendu par la société GAF.

**MOWIOL 40-88** : Alcool polyvinylique vendu par la société HOECHST

**PVP/VA S 630** : Copolymère vinylpyrrolidone/acétate de vinyle ( 60/40 ) vendu par la société GAF.

**CELQUAT LOR** : Copolymère d'hydroxyéthylcellulose et de chlorure de diallyldiméthylammonium vendu par la société NATIONAL STARCH.

**GAFQUAT 734** : Copolymère polyvinylpyrrolidone/Méthacrylate de diméthylaminoéthyl quaternisé par du sulfate de diéthyle ayant un poids moléculaire de 100.000 vendu par la société GAF à 50% de matière active dans l'éthanol.

**FLUID DC 200**(12500 Cst) : Huile polydiméthylsiloxane ( viscosité 125 $cm^2/s$, 12500 Cst à 25°C) vendue par la société DOW CORNING.

**ABIL AV 1000** : Huile polyméthylphénylsiloxane ( viscosité 10 $cm^2/s$, 1000 Cst à 25°C) vendue par la société GOLDSCHMIDT.

**SILBIONE 70047V5000** : Huile polydiméthylsiloxane ( viscosité 50 $cm^2/s$, 5000 Cst à 25°C) vendue par la société RHONE POULENC.
**SILBIONE 70045V5** : Décaméthylcyclopentasiloxane vendu par la société RHONE POULENC.

EXEMPLES 1-10 :

On prépare les compositions suivantes (teneurs pondérales en g de matière active) :

| EXEMPLES | 1 | 2 | 3 | 4 | 5 | 6 | 7 | 8 | 9 | 10 |
|---|---|---|---|---|---|---|---|---|---|---|
| **Silicones** | | | | | | | | | | |
| Silbione 70047V5000 ( R.P. ) | 0,05 | | | | 0,05 | 0,05 | 0,05 | 0,05 | | |
| Silbione 70045V5 ( R.P. ) | | 0,05 | | | | | | | 0,05 | |
| FLUID DC200 (12500Cst) ( D.C. ) | | | 0,05 | | | | | | | |
| PPMS ABIL AV 1000 (Goldschmidt) | | | | 0,05 | | | | | | 0,05 |
| **PA** | | | | | | | | | | |
| Gantrez ES 425 ( GAF ) | 0,2 | | | | | | | | 0,2 | 0,2 |
| **PC** | | | | | | | | | | |
| CELQUAT LOR N.S. | | 0,2 | | | | | | | | |
| GAFQUAT 734 50%MA ( GAF ) | | | | | 0,2 | | | | | |
| **PNI** | | | | | | | | | | |
| PVP/VA S 630 ( GAF ) | | | | | | 0,2 | | | | |
| MOWIOL 40-88 ( HOECHST ) | | | 0,2 | | | | | | | |
| **PAM** | | | | | | | | | | |
| Dérivé de chitosane P 1 | | | | 0,2 | | | 0,2 | | | |
| Kératine sulfonique | | | | | | | | 0,2 | | |
| **Composition A** | 90 | 90 | 90 | 90 | 90 | 90 | 90 | 90 | 90 | 90 |
| Hydroxyde de sodium | 0,035 | | | | | | | | 0,035 | 0,035 |
| Acide lactique | | | | 0,19 | | | 0,19 | | | |
| Eau qsp | 100 | 100 | 100 | 100 | 100 | 100 | 100 | 100 | 100 | 100 |

On effectue cette préparation en ajoutant la quantité requise de polymère filmogène et de silicone à la microdispersion de composition A, qui a la teneur indiquée ci-après. Finalement, on ajoute, le cas échéant, l'acide lactique ou l'hydroxyde de sodium.

Composition A:

Composition A :

| | | | |
|---|---|---|---|
| Cire autoémulsionnable vendue sous la dénomination Cire autolustrante OFR par la société TISCO | | | 12 g |
| Triéthanolamine | qs | pH 5 | |
| Conservateurs | qs | | |
| Eau | qsp | | 100 g |

On fait fondre la cire OFR à 90-95°C sous agitation puis on ajoute progressivement sous agitation modérée 70% de la quantité totale d'eau portée à 90°C et renfermant un conservateur. On laisse revenir le mélange à 30°C sous agitation douce , on ajuste le pH avec la triéthanolamine ,on ajoute des conservateurs puis on complète à 100 g par l'eau restante.

Exemple 11:

De façon analogue, on a préparé la composition suivante :

- Composition B 18 g
- Huile 71 615 V 300 RP 0,5 g
  (Polydiméthylsiloxane à groupement hydroxypropylé de PM 900)
- Hydroxyéthylcellulose réticulée à l'épichlorhydrine quaternisée par la tiréthanolamine vendue sous le nom JR 400 par la Société GAF 0,3 g
- Monolaurate de sorbitane polyoxyéthyléné à 20 moles d'oxyde d'éthylène (OE) 3,0 g
- Hydroxyéthylcellulose 1,0 g
- Eau qsp 100 g

Le pH est ajusté à 4 avec l'acide lactique.

La Composition B a la teneur suivante :

- Cire de carnauba 30 g
- Monostéarate de glycérol polyoxyéthyléné à 30 moles d'OE vendu par la Société GOLDSCHMIDT sous la dénomination Tagat S 7,5 g
- Conservateur 0,2 g
- Eau qsp 100 g

TESTS SUR CHEVEUX

Ces tests ont été effectués avec les compositions des exemples 1 à 10 ainsi que sur des compositions analogues en omettant soit le polymère filmogène, soit la silicone. Les résultats sont comparés à ceux obtenus avec la "microdispersion nue" (sans polymère filmogène ni silicone) qui sert de témoin.

Les procédures utilisées sont indiquées ci-après.

TEST DE DOUCEUR

Mise en oeuvre

On étale 0,25g de la composition étudiée sur des mèches de cheveux naturels de 5g. On peigne la mèche et la sèche au casque pendant 15 min. On laisse la mèche refroidir et on procède à l'évaluation de la douceur.

Evaluation

On évalue la douceur de la mèche considérée par toucher comparatif par rapport à la mèche témoin.

Une même mèche ne sert au plus qu'à deux évaluations. On s'affranchit ainsi des problèmes de modification de la douceur induite par des manipulations successives des mèches.

Le test est effectué sur un panel composé de 5 testeurs au minimum.

TEST DE NERVOSITE

Mise en oeuvre

On étale 0,25g de la composition étudiée sur une mèche de cheveux de 2,5g ayant 25cm de longueur. On peigne la mèche et l'enroule sur un bigoudi de 2cm de diamètre (nombre de spires=4). On sèche la mèche, montée sur bigoudi, au casque pendant 15 min. On laisse la mèche refroidir avant de la dérouler et de l'installer sur l'appareil décrit ci-dessous, permettant l'appréciation de la nervosité.

Appareillage

On utilise pour cette appréciation un appareil qui impose à des mèches, fixées par leurs deux extrémités audit appareil, un mouvement vertical alternatif de course et vitesse connues dans des conditions opératoires reproductibles suivantes : 100 cycles à raison de 50 cycles/min.

A l'issue de ces contraintes, l'extrémité inférieure de la mèche est libérée et l'on procède alors à une évaluation visuelle et tactile de la nervosité.

Chaque composition est évaluée par 5 ou 6 testeurs.

L'évaluation visuelle permet de sélectionner les mèches encore bouclées et non détendues qui sont soumises alors à une apréciation tactile consistant à évaluer au toucher "l'effet ressort" des mèches bouclées.

RESULTATS

Les compositions selon l'invention ont été jugées supérieures en douceur et nervosité à la microdispersion nue (sans polymère filmogène ni silicone) par au moins 80% des testeurs. Les compositions des exemples 1 à 4 ont été jugées supérieures par 100% des testeurs.

## Revendications

**1.** Composition cosmétique pour cheveux comprenant au moins un polymère filmogène et au moins une silicone incorporés dans un support constitué essentiellement d'une dispersion de cire dans un véhicule liquide aqueux dont la phase dispersée est une microdispersion stable de particules de dimensions inférieures à 500 nm, lesdites particules étant constituées essentiellement d'une cire ou d'un mélange de cires, ladite cire ou ledit mélange de cires ayant un point de fusion finissante supérieur à 60°C et inférieur à 100°C et étant capable de former une microdispersion telle que définie ci-dessus, ladite composition contenant, en poids, de 0,1 à 40 % de cire et une quantité suffisante d'au moins un agent émulsionnant.

**2.** Composition selon la revendication 1, caractérisée par le fait que la cire comprend au moins 50% en poids d'une cire choisie parmi la cire de Carnauba, la cire de Candelilla, la cire d'Alfa et leurs mélanges.

**3.** Composition selon la revendication 1, caractérisée par le fait que la cire est choisie parmi la cire de Carnauba, la cire de Candelilla, la cire d'Alfa et leurs mélanges.

**4.** Composition selon l'une quelconque des revendications précédentes, caractérisée par le fait que la proportion pondérale de cire peut varier de 0,1 à 20%, et en particulier de 1 à 15%.

**5.** Composition selon l'une quelconque des revendications précédentes, caractérisée par le fait que la proportion pondérale de cire peut varier de 1 à 10%.

**6.** Composition selon l'une quelconque des revendications précédentes, caractérisée par le fait que ledit agent émulsionnant est un agent tensio-actif anionique ou non ionique.

7. Composition selon l'une quelconque des revendications précédentes, caractérisée par le fait que la proportion pondérale d'agent émulsionnant peut varier de 0,01 à 25% en poids, et en particulier de 0,1 à 10% en poids.

8. Composition selon l'une quelconque des revendications précédentes, caractérisée par le fait que le rapport pondéral cire/émulsionnant est compris dans la gamme de 1 à 20, et en particulier de 2 à 10.

9. Composition selon l'une quelconque des revendications précédentes, caractérisée par le fait que la proportion pondérale de silicone peut varier de 0,01 à 1%, et en particulier de 0,02 à 0,5%.

10. Composition selon l'une quelconque des revendications précédentes, caractérisée par le fait que la proportion pondérale de polymère filmogène peut varier de 0,1 à 2% et en particulier de 0,1 à 1%.

11. Composition selon l'une quelconque des revendications précédentes, caractérisée par le fait que le rapport pondéral polymère filmogène/silicone est supérieur à 0,25 et inférieur à 8.

12. Composition selon la revendication précédente, caractérisée par le fait que ledit rapport est supérieur à 0,5 et inférieur à 6.

13. Procédé de traitement cosmétique, caractérisé par le fait que l'on applique sur les cheveux une quantité efficace d'une composition telle que définie dans l'une quelconque des revendications précédentes.

## Patentansprüche

1. Kosmetische Zusammensetzung für die Haare, enthaltend mindestens ein filmbildendes Polymer und mindestens ein in einem im wesentlichen aus einer Wachsdispersion in einem wäßrigen Flüssigträger inkorporiertes Silikon, wobei die dispergierte Phase eine stabile Mikrodispersion von Teilchen kleiner 500 nm ist, und die Teilchen im wesentlichen aus einem Wachs oder einer Mischung von Wachsen bestehen und das Wachs oder die Mischung der Wachse einen Endschmelzpunkt von mehr als 60 °C und weniger als 100 °C aufweist/aufweisen, und in der Lage ist/sind, eine wie vorstehend definierte Mikrodispersion zu bilden, wobei die Zusammensetzung 0,1 bis 40 Gew.-% Wachs und eine ausreichende Menge mindestens eines Emulgiermittels enthält.

2. Zusammensetzung nach Anspruch 1, dadurch gekennzeichnet, daß das Wachs mindestens 50 Gew.-% eines Wachses, ausgewählt aus Carnauba-Wachs, Candelilla-Wachs, Alfa-Wachs und ihre Mischungen, enthält.

3. Zusammensetzung nach Anspruch 1, dadurch gekennzeichnet, daß das Wachs ausgewählt ist aus Carnauba-Wachs, Candelilla-Wachs, Alfa-Wachs und ihren Mischungen.

4. Zusammensetzung nach einem der vorhergehenden Ansprüche, dadurch gekennzeichnet, daß der Gewichtsanteil des Wachses zwischen 0,1 bis 20 %, und insbesondere zwischen 1 und 15 %, variieren kann.

5. Zusammensetzung nach einem der vorhergehenden Ansprüche, dadurch gekennzeichnet, daß der Gewichtsanteil des Wachses zwischen 1 und 10 % variiert.

6. Zusammensetzung nach einem der vorhergehenden Ansprüche, dadurch gekennzeichnet, daß das Emulgiermittel ein anionisches oder nicht-ionisches oberflächenaktives Mittel ist.

7. Zusammensetzung nach einem der vorhergehenden Ansprüche, dadurch gekennzeichnet, daß der Gewichtsanteil des Emulgiermittels von 0,01 bis 25 Gew.-% und insbesondere zwischen 0,1 bis 10 Gew.-% variieren kann.

8. Zusammensetzung nach einem der vorhergehenden Ansprüche, dadurch gekennzeichnet, daß das Gewichtsverhältnis Wachs/Emulgiermittel im Bereich von 1 bis 20 und insbesondere von 2 bis 10 liegt.

9. Zusammensetzung nach einem der vorhergehenden Ansprüche, dadurch gekennzeichnet, daß der Gewichtsanteil des Silikons zwischen 0,01 und 1 % und insbesondere zwischen 0,02 und 0,5 % variieren kann.

10. Zusammensetzung nach einem der vorhergehenden Ansprüche, dadurch gekennzeichnet, daß der Gewichtsanteil des filmbildenden Polymers zwischen 0,1 und 2 % und insbesondere zwischen 0,1 und 1 % variieren kann.

11. Zusammensetzung nach einem der vorhergehenden Ansprüche, dadurch gekennzeichnet, daß das Gewichtsverhältnis filmbildendes Polymer/Silikon größer als 0,25 und kleiner als 8 ist.

12. Zusammensetzung nach dem vorhergehenden Anspruch, dadurch gekennzeichnet, daß das Verhältnis größer als 0,5 und kleiner als 6 ist.

13. Verfahren zur kosmetischen Behandlung, dadurch gekennzeichnet, daß man auf die Haare eine wirksame Menge einer Zusammensetzung, wie in einem der vorhergehenden Ansprüche definiert, aufbringt.

## Claims

1. Cosmetic hair composition comprising at least one film-forming polymer and at least one silicone incorporated into a carrier essentially consisting of a dispersion of wax in an aqueous liquid vehicle of which the dispersed phase is a stable microdispersion of particles of less than 500 nm in size, the said particles essentially consisting of a wax or mixture of waxes, the said wax or the said mixture of waxes having a final melting point greater than 60°C and less than 100°C and being capable of forming a microdispersion as defined above, the said composition containing, by weight, from 0.1 to 40 % wax and sufficient quantity of at least one emulsifying agent.

2. Composition according to Claim 1, characterized by the fact that the wax comprises at least 50 % by weight of a wax chosen from Carnauba wax, Candilla wax, Esparto wax and mixtures thereof.

3. Composition according to Claim 1, characterized by the fact that the wax is chosen from Carnauba wax, Candelilla wax, Esparto wax and mixtures thereof.

4. Composition according to any one of the preceding claims, characterized by the fact that the proportion by weight of wax may range from 0.1 to 20 %, and in particular from 1 to 15 %.

5. Composition according to any one of the preceding claims, characterized by the fact that the proportion by weight of wax may range from 1 to 10 %.

6. Composition according to any one of the preceding claims, characterized by the fact that the said emulsifying agent is an anionic or non-ionic surface-active agent.

7. Composition according to any one of the preceding claims, characterized by the fact that the proportion by weight of emulsifying agent may range from 0.01 to 25 % by weight, and in particular from 0.1 to 10 % by weight.

8. Composition according to any one of the preceding claims, characterized by the fact that the wax/emulsifier weight ratio is in the range of 1 to 20, and in particular 2 to 10.

9. Composition according to any one of the preceding claims, characterized by the fact that the proportion by weight of silicone may range from 0.01 to 1 %, and in particular from 0.02 to 0.5 %.

10. Composition according to any one of the preceding claims, characterized by the fact that the proportion by weight of film-forming polymer may range from 0.1 to 2 % and in particular from 0.1 to 1 %.

11. Composition according to any one of the preceding claims, characterized by the fact that the film-forming polymer/silicone weight ratio is greater than 0.25 and less than 8.

12. Composition according to the preceding claim, characterized by the fact that the said ratio is greater than 0.5 and less than 6.

13. Process for cosmetic treatment, characterized by the fact that an effective quantity of a composition as defined in any one of the preceding claims is applied to the hair.